# EUROPEAN PATENT SPECIFICATION

(11) **EP 2 014 775 B1**
(45) Date of publication and mention of the grant of the patent: **05.11.2014**
(21) Application number: 07119610.9
(22) Date of filing: 30.10.2007
(51) Int. Cl.: C12Q 1/68

(54) **Method for the detection of bacterial species of the genera Anaplasma/Ehrlichia and Bartonella**
Verfahren zum Nachweis von Bakterienarten der Gattungen Anaplasma/Ehrlichia und Bartonella
Procédé de détection des espèces bactériennes de Anaplasma/Ehrlichia et Bartonellose

(30) Priority: 29.06.2007 ES 200701830
(43) Date of publication of application: 14.01.2009
(73) Proprietor: Instituto de Salud Carlos III, 28029 Madrid (ES)
(72) Inventor: Anda Fernández, Pedro, 28029, Madrid (ES); Gil Gil, Horacio, 28029, Madrid (ES); Escudero Nieto, Raquel, 28029, Madrid (ES); Jado García, Isabel, 28029, Madrid (ES); Rodríguez Moreno, Isabel, 28029, Madrid (ES)
(74) Representative: Pons Ariño, Angel

(56) References cited:
- EP-A- 1 895 015
- DATABASE Geneseq [Online] 22 March 2007 (2007-03-22), "Anaplasma/Ehrlichia 16S rRNA PCR primer 16S/AE-R, SEQ:2." XP007906164 retrieved from EBI accession no. GSN:AEM74242 Database accession no. AEM74242
- DATABASE EMBL [Online] 12 December 2006 (2006-12-12), "ia217f01.x1 mouse taste receptor cell, subtracted, normalized Mus musculus cDNA clone ia217f01 3', mRNA sequence." XP007906165 retrieved from EBI accession no. EMBL:EH102208 Database accession no. EH102208
- DATABASE EMBL [Online] 27 September 2002 (2002-09-27), "Sequence 17 from patent US 6429292." XP007906166 retrieved from EBI accession no. EMBL:AR222418 Database accession no. AR222418
- DATABASE EMBL [Online] 1 July 2006 (2006-07-01), "ELE00014520 High Light Acclimated Euglena gracilis cDNA, mRNA sequence." XP007906167 retrieved from EBI accession no. EMBL:EC666906 Database accession no. EC666906
- DATABASE EMBL [Online] 23 February 2001 (2001-02-23), "2M0103K21F Mouse 10kb plasmid UUGC1M library Mus musculus genomic clone UUGC2M0103K21 F, DNA sequence." XP002505698 retrieved from EBI accession no. EMBL:AZ827153 Database accession no. AZ827153
- DATABASE EMBL [Online] 1 September 2006 (2006-09-01), "MSAM265146_3285_1923 LCM-dissected maize shoot apical meristem cDNA Zea mays cDNA, mRNA sequence." XP007906170 retrieved from EBI accession no. EMBL:DW962898 Database accession no. DW962898
- DATABASE EMBL [Online] 21 July 2005 (2005-07-21), "Anaplasma platys isolate Santiago 7 16S ribosomal RNA gene, partial sequence." XP007906171 retrieved from EBI accession no. EMBL:DQ125261 Database accession no. DQ125261
- DATABASE EMBL [Online] 22 June 2006 (2006-06-22), "271859_1875_3153 LNCAP + R1881 synthetic Androgen human prostate library Homo sapiens cDNA similar to ENSG00000156508¦ENST00000358190, mRNA sequence." XP007906173 retrieved from EBI accession no. EMBL:EC444979 Database accession no. EC444979
- DATABASE EMBL [Online] 10 July 1991 (1991-07-10), "Rhizobium tropici 16s ribosomal RNA gene, partial sequence." XP007906174 retrieved from EBI accession no. EMBL:M64319 Database accession no. M64319
- DATABASE EMBL [Online] 27 February 2006 (2006-02-27), "Oryza sativa Japonica Group cDNA, clone: J01B3058J18M3, 3'end of J013058J18." XP007906175 retrieved from EBI accession no. EMBL:CI320977 Database accession no. CI320977
- DATABASE EMBL [Online] 10 February 2007 (2007-02-10), "EB3RODY01AXL6Y 8-day Arabidopsis seedlings, aerial tissues Arabidopsis thaliana cDNA, mRNA sequence." XP007906176 retrieved from EBI accession no. EMBL:EL192246 Database accession no. EL192246
- DATABASE EMBL [Online] 2 December 2003 (2003-12-02), "I044P93 Populus senescing leaves cDNA library Populus tremula cDNA clone I044P93 5', mRNA sequence." XP007906177 retrieved from EBI accession no. EMBL:CK108605 Database accession no. CK108605
- DATABASE EMBL [Online] 10 February 2007 (2007-02-10), "CCEM9476.b1_G18.ab1 CCE(LMS) endive Cichorium endivia cDNA clone CCEM9476, mRNA sequence." XP002505697 retrieved from EBI accession no. EMBL:EL364957 Database accession no. EL364957
- DATABASE EMBL [Online] 1 September 2006 (2006-09-01), "MSAM138073_2646_1817 LCM-dissected maize shoot apical meristem cDNA Zea mays cDNA, mRNA sequence." XP007906179 retrieved from EBI accession no. EMBL:DW906780 Database accession no. DW906780
- DATABASE EMBL [Online] 24 February 2006 (2006-02-24), "6626350 CERES-AL45 Arabidopsis thaliana cDNA clone 1190525 5', mRNA sequence." XP007906180 retrieved from EBI accession no. EMBL:DR275525 Database accession no. DR275525
- DATABASE EMBL [Online] 10 February 2007 (2007-02-10), "EB3RODY02IGSJQ 8-day Arabidopsis seedlings, aerial tissues Arabidopsis thaliana cDNA, mRNA sequence." XP007906181 retrieved from EBI accession no. EMBL:EL262802 Database accession no. EL262802
- DATABASE EMBL [Online] 4 April 2004 (2004-04-04), "SAK7H08 Flanking Sequence Tag of Oryza sativa T-DNA insertion lines Oryza sativa (japonica cultivar-group) genomic, genomic survey sequence." XP007906182 retrieved from EBI accession no. EMBL:CL520947 Database accession no. CL520947
- DATABASE EMBL [Online] 5 October 2000 (2000-10-05), "1M0165D21F Mouse 10kb plasmid UUGC1M library Mus musculus genomic clone UUGC1M0165D21 F, DNA sequence." XP007906183 retrieved from EBI accession no. EMBL:AZ399495 Database accession no. AZ399495
- DATABASE Geneseq [Online] 19 July 2002 (2002-07-19), "Sphingomonas paucimobilis PCR primer PAUC1." XP007906184 retrieved from EBI accession no. GSN:AAL37761 Database accession no. AAL37761
- SCHOULS L M ET AL: "Detection and identification Ehrlichia, Borrelia burgdorferi Sensu Lato, and Bartonella species in dutch Ixodes ricinus ticks" JOURNAL OF CLINICAL MICROBIOLOGY, WASHINGTON, DC, US, vol. 37, no. 7, 1 July 1999 (1999-07-01), pages 2215-2222, XP003005400 ISSN: 0095-1137
- MASSUNG R F ET AL: "Comparison of PCR assays for detection of the agent of human granulocytic ehrlichiosis, Anaplasma phagocytophilum" JOURNAL OF CLINICAL MICROBIOLOGY, WASHINGTON, DC, US, vol. 41, no. 2, 1 February 2003 (2003-02-01), pages 717-722, XP009108054 ISSN: 0095-1137
- MARTÍNEZ-ROMERO E ET AL: "Rhizobium tropici, a novel species nodulating Phaseolus vulgaris L. beans and Leucaena sp. trees." INTERNATIONAL JOURNAL OF SYSTEMATIC BACTERIOLOGY JUL 1991, vol. 41, no. 3, July 1991 (1991-07), pages 417-426, XP009108132 ISSN: 0020-7713

## Description

The present invention, relating to a method for the detection and identification of bacterial species belonging to the genera Anaplasma/Ehrlichia and Bartonella, is a patent of addition to the Spanish patent application with publication number 2.264.642. In addition to this method, the invention also provides the primers and probes required for its application.

### BACKGROUND

To date, 200 zoonotic diseases (bartonelosis, leptospirosis, Lyme borreliosis...) which affect humans and represent one of the main causes of death and entail substantial economic loss in third world countries, have been described. Coexistence with animals, lack of sanitary infrastructure and low cultural level continue to be the main allies of these diseases.

In the same manner, certain types of zoonosis which are widespread in third world countries are now thriving in industrialized countries as a consequence of population increases in urban and periurban areas, in addition to increased movement of animals across international borders. These circumstances, amongst others, entail the risk of introducing exotic diseases into our environment.

Additionally, the frequent findings of arthropods infected by more than one of the pathogens included in the present invention, increases the possibility of being transmitted more than one zoonotic disease in a single sting. As a result, hospitalizations due to medical profiles produced by contact with animals or arthropods, such as mosquitoes, ticks, fleas, lice, mites, etc., which act as vectors or pathogen reservoirs, is becoming increasingly common. Said medical profiles, due to their high degree of similarity, do not allow a fast and reliable identification of the pathogenous agent, so that specific and fast treatment is not possible and is occasionally administered too late. This undoubtedly justifies the need for a comprehensive method to detect and identify bacterial species that cause zoonosis.

To date, the molecular diagnosis methods available are basically limited to the detection of pathogens based on antibody technology. This type of analysis, generally retrospective and with low sensitivity levels, is normally of little use to treating diseases in acute-phase states.

Another alternative for the detection and identification of pathogens is based on the application of culture mediums. These types of techniques are scarcely applicable to certain species of genera such as Bartonella and Anaplasma/Ehrlichia, due to the fact that said species do not normally grow in regular culture mediums and may even require cellular cultures. As a result, these methodologies are isolated from regular practice in hospital microbiology laboratories. One of the most effective alternatives to these types of methodologies is the direct analysis of genetic material, based on PCR technology. Said technology, while being highly effective, is greatly limited by the difficulty in finding specific markers or regions, in addition to primers and probes, which ensure reliable sample analysis.

A paper has recently been published (Blaskovic D. et al. 2005. FEMS Microbiology Letters 243:273-8) which describes a method based on ribosomal DNA analysis, although it uses universal primers which amplify the genetic material of both target and non-target bacteria, due to which its sensitivity is substantially reduced.

Other methods, such as those described by American patents US 6,300,072 and 6,518,020, are capable of detecting and identifying bacteria of the genus ***Bartonella*** by using the same region (16S-23S), even though the number of species within this genus has increased substantially since said patents were filed and their approximation, which consists of discriminating between species according to the size of the amplicon obtained during PCR, is not useful for certain known species within the same genus which are similar in size to the amplified fragment.

Schoulds L.M. et al. 1999 (Journal of clinical Microbiology, 37(7): 2215-2222) disclose a method for simultaneous detection of bacterial species that causes zoonosis belonging to the genera Ehrlichia and Bartonella by means of primers and probes that amplify regions within 16S sRNA gene. Massung R.F. et al. 2003 (Journal of Clinical Microbiology, 41(2): 717-722) disclose a comparison of different PCR assays in order to assess its analytical sensitivity for detecting *Anaplasma phagocytophilum.* The assays which provide the highest sensitivity and specifity for detecting *Anaplasma phagocytophilum-*infected cells were the 16S-nested assay and the msp2 assay.

### BRIEF DESCRIPTION OF THE INVENTION

The present invention proposes the use of genes 16S and msp2 and intergenic space 16S-23S for the detection and identification of the different bacterial species and groups of species that cause zoonosis, improving the previously described procedures based on its capacity to specifically detect a substantial number of bacterial species using probes and primers with high sensitivity levels.

Therefore, the present invention solves the problem of the tediousness and complexity of detecting a high number of bacteria that cause zoonosis which can be clinically and/or epidemiologically indistinguishable, through the development of a detection method and Kit based on PCR technology. Specifically, the invention allows the analysis of different regions of bacterial DNA belonging to the genera Anaplasma/Ehrlichia and Bartonella in order to identify both genus and species, as shown in the following table (Table 1).

In most cases, the species identified correspond to cultured species and, in others, correspond to species isolated for the first time (non-cultured species). Said species have been obtained from samples of *Meles meles* (badger), *Ixodes ricinus* and *Apodemus sylvaticus* species and have been characterized by the sequences AJ269792, SEQ ID NO:44-46, as shown in table 3.

| | |
|---|---|
| ***BACTERIAL SPECIES*** | **GENE** |

| ***Anaplasma*/*Ehrlichia*** | |
|---|---|
| *A. phagocytophilum msp2* | msp2 |
| *Ehrlichia ruminantium* | 16S |
| *E. sennetsu* | 16S |
| *E. risticii* | 16S |
| *E. muris* | 16S |
| *A. platys* | 16S |
| *E. canis*/*E. ovina* | 16S |

| ***Bartonella*** | |
|---|---|
| Generic | 16S |
| *B. talpae* | 16S-23S |
| *Bartonella sp. ** | 16S-23S |
| *B. phoceensis* | 16S-23S |
| *B. rattimasiliensis* | 16S-23S |
| *Bartonella sp. detected in Apodemus sylvaticus* | 16S-23S |
| *B. rochalimae* | 16S-23S |
| *Bartonella sp. detected in badger* | 16S-23S |
| *Bartonella sp** detected in Ixodes ricinus* | 16S-23S |
| *Bartonella sp detected in Ixodes ricinus* | 16S-23S |

| | |
|---|---|
| * B. chomeli/schoenbuchensis/capreoli/birtlesii | |

### BRIEF DESCRIPTION OF THE DRAWINGS

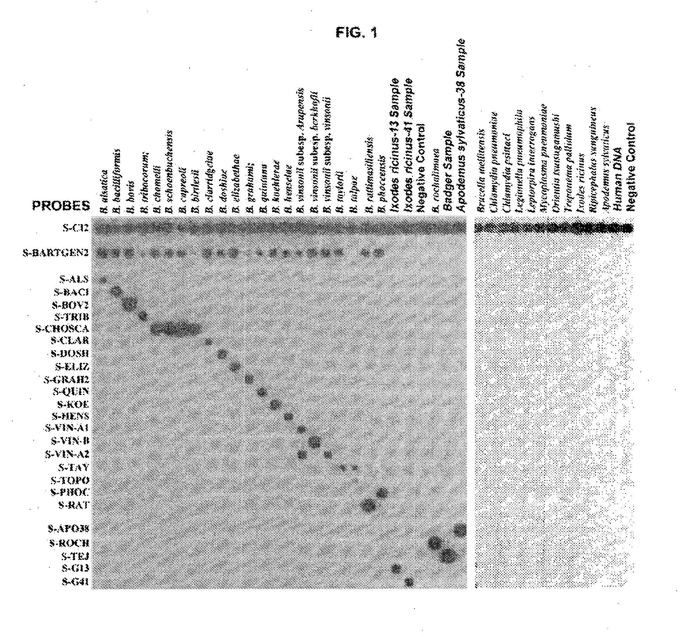

**Figure 1****.-** This figure represents two hybridization membranes for the validation of primers and probes used to detect the Bartonella genus. In the membrane on the left we can observe the absence of cross-react between the different probes within the genus. In the membrane on the right we can observe the absence of cross-react in samples which do not contain the Bartonella genus. The S-CI2 probe refers to the IAC probe.

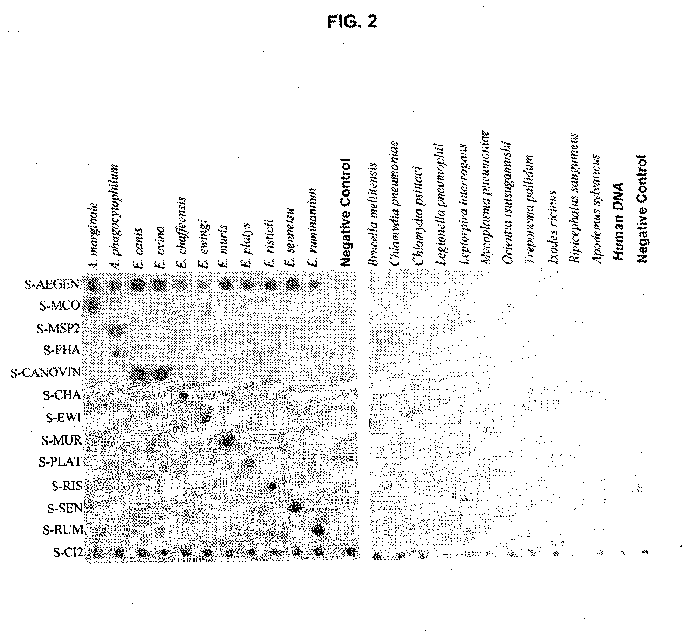

**Figure 2****.-** This figure represents two hybridization membranes for the validation of primers and probes used to detect the genera Anaplasma/Ehrlichia. In the membrane on the left we can observe the absence of crossed reactivity between the different probes within the genus. In the membrane on the right we can observe the absence of cross-reactivity in samples which do not contain the genera Anaplasma/Ehrlichia. The S-CI2 probe refers to the IAC probe.

### DETAILED DESCRIPTION OF THE INVENTION

According to a first aspect of the invention, said invention relates to a method (hereinafter, method of the invention) for the simultaneous and specific identification of zoonosis-causing bacteria belonging to the genera *Anaplasma*/*Ehrlichia* and to the genus *Bartonella* comprising the following steps:
a. Placing the sample under analysis in contact with a reaction mixture containing primers that specifically amplify the sequences SEQ ID NO: 1-7 of the *Anaplasma*/*Ehrlichia* species and sequences SEQ ID NO: 8-17 of *Bartonella* species, or their respective complementary sequences,
b. Amplifying simultaneously the sequences of step (a) by means of polymerase chain reaction,
c. Identifying the amplified sequences of step (b) by using the probes consisting of SEQ ID NO: 26-42, or their complementary sequences, to hybridise with sequences SEQ ID NO: 1-17, and
d. Detecting the presence or absence of zoonosis-causing bacteria by associating the identified sequences of step (c) to a bacterial species belonging to the genera *Anaplasma*/*Ehrlichia* or to the genus *Bartonella,*
wherein the bacterial species belonging to the genera *Anaplasma*/*Ehrlichia* are selected from the list consisting of *A. phagocytophilum, E. ruminantium, E, sennetsu, E. risticii, E. muris, A. platys, E. canis* and *E. Ovina.*

The primers required to apply the method of the invention may be designed by means of multiple alignment with the sequences comprising SEQ ID NO:1-17 using computer programs such as CLUSTAL X, and allow the identification of highly preserved regions that will act as a mould for primer design, which must subsequently be validated empirically.

According to a preferred embodiment of this aspect of the invention, the primers are capable of hybridizing with different nucleotide regions of the genes 16S, msp2 and intergenic space 16S-23S (tables 2 and 3), said primers being selected from the SEQ ID NO: 18-25 group or their complementary sequences and being capable of amplifying SEQ ID NO:1-17, or their complementary sequences in a simultaneous manner. These primers, in addition to simplifying the method, have the advantage of low or null reactivity with respect to samples of other species (see table 5).

The detection of sequences SEQ ID NO:1-17, or their complementary sequences, may be carried out based on well-known methodologies within the art using probes, said probes being capable of hybridizing between the positions of genes 16S, msp2 and intergenic space 16S-23S, as indicated in tables 2 and 3, and consisting of the sequences SEQ ID NO:26-42 or their complementary sequences.

In a particular embodiment, the method ot the invention further comprises the primers with sequence SEQ ID NO: 51-52 in order to amplify the internal amplification control THC synthase gen of *Cannabis sativa.*

In another particular embodiment, the probes of step (c) of the method of the invention further comprise the probe with sequence SEQ ID NO: 50 capable of hybridizing with the sequence SEQ ID NO: 49 of the internal amplification control.

The invention also discloses primers capable of amplifying the sequences selected from the group comprising SEQ ID NO:1-17, 47 and their complementary sequences. Said primers shall be capable of hybridizing between the nucleotide positions of genes 16S, msp2 and intergenic space 16S-23S, as indicated in tables 2 and 3 (column 3). According to the invention, the primers are selected from the group consisting of the sequences SEQ ID NO: 18-25 and their complementary sequences.

Another aspect of the invention relates to probes capable of specifically detecting any of the bacterial species and genera, as indicated in tables 2 and 3 (column 6), said probes being capable of hybridizing between nucleotide positions of genes 16S, msp2 and intergenic space 16S-23S, as indicated in tables 2 and 3. Said probes, the sequence of which consists of SEQ ID NO: 26-32 of the *AnaplasmalEhrlichia* species *A. phagocytophilum, E. ruminantium, E, sennetsu, E. risticii, E. muris, A. platys, E. canis* and *E. ovina,* and sequences SEQ ID NO: 33-42 of *Bartonella* species, or their complementary sequences, are capable of hybridizing with the sequences SEQ ID NO: 1-17 and/or with their respective complementary sequences. Hereinafter, these will be referred to as probes of the invention.

Another aspect of the invention relates to an analysis kit for the identification of any of the bacterial genera or species, as indicated in tables 2 and 3, where said kit comprises the probes of the invention and, in a particular embodiment, further comprises the primers with sequences SEQ ID NO: 18-25. Additionally, this kit may include all the reactive agents, buffers, supports, etc. required for its development, without limitation.

In another aspect, the invention relates to the use of the kit for the simultaneously specific identification of zoonosis-causing bacteria belonging to the genera *Anaplasma*/*Ehrlichia* and to the genus *Bartonella,* wherein the bacterial species belonging to the genera *Anaplasma*/*Ehrlichia* are selected from the list consisting of *A. phagocytophilum, E. ruminantium, E, sennetsu, E. risticii, E. muris, A. platys, E. canis* and *E*. *Ovina.*

| **TABLE 2. Detection and identification of species belonging to the genera Anaplasma/Ehrlichia** | | | | | |
|---|---|---|---|---|---|
| **ORGANISM** | **GENE** | **PRIMER** | **PROBES** | **SEQUENCES** | **POSITION** |
| **Anaplasma (Ehrlichia)** | | | | | |
| **A. phagocytophilum** | msp2 | SEQ ID NO:18 (EF143812 (1-22)) | MSP2 (SEQ ID NO:26) | SEQ ID NO:1 | EF143812 (223-243) |
| | | SEQ ID NO:19 (EF143812 (313-334) | | | |
| **Generic** | 16S | SEQ ID NO:20. (U02521 (9-30) | AEGEN (SEQ ID NO:48) | SEQ ID NO:47 | U02521 (38-57) |
| | | SEQ ID NO:21 (U02521 (109-86) | | | |
| **Ehrlichia ruminantium** | 16S | SEQ ID NO:21 | S-RUM (SEQ ID NO:27) | SEQ ID NO:2 | DQ640401 (23-45) |
| | | SEQ ID NO:21 | | | |
| **E. sennetsu** | 16S | SEQ ID NO:20. | S-SEN (SEQ ID NO:28) | SEQ ID NO:3 | M73225 (46-63) |
| | | SEQ ID NO:21 | | | |
| **E. risticii** | 16S | SEQ ID NO:20. | S-RIS (SEQ ID NO:29) | SEQ ID NO:4 | AY005439 (46-65) |
| | | SEQ ID NO:21 SEQ | | | |
| **E. muris** | 16S | SEQ ID NO:20. | (SEQ ID NO:30) | SEQ ID NO:5 | AY587608 (16-37) |
| | | SEQ ID NO:21 | | | |
| **A. platys** | 16S | SEQ ID NO:20. | S-PLA (SEQ ID NO:311) | SEQ ID NO:6 | EF139459 (53-75) |
| | | SEQ ID NO:21 | | | |
| **E. canis/E. ovina** | 16S | SEQ ID NO:20. | S-CANOVIN (SEQ ID NO:32) | SEQ ID NO:7 | EF011111 (14-37) |
| | | SEQ ID NO:21 | | | |

| **TABLE 3. Detection and identification of species belonging to the genus Bartonella** | | | | | |
|---|---|---|---|---|---|
| **ORGANISM** | **GENE** | **PRIMER** | **PROBES** | **SEQUENCES** | **POSITION** |
| **Bartonella** | | | | | |
| **Generic** | 16S | SEQ ID NO:22 (AJ223780 (961-979)) | BARTGEN2 (SEQ ID NO:33) | SEQ ID NO:8 | AJ223780 (1054-1075) |
| | | SEQ ID NO:23 (AJ223780 (1376-1398)) | | | |
| ***B**. **talpae*** | 16S-23S | SEQ ID NO:24 (AY116638 (455-478)) | S-TOPO (SEQ ID NO:34) | SEQ ID NO:9 | SEQ ID NO: 43 (90-113) |
| | | SEQ ID NO:25 (724-743 (AJ269786)) | | | |
| ***B**. **phoceensis*** | 16S-23S | SEQ ID NO:24 | S-PHO (SEQ ID NO:35) | SEQ ID NO:10 | AY515123 (659-679) |
| | | SEQ ID NO:25 | | | |
| ***B**. **rattimasiliensis*** | 16S-23S | SEQ ID NO:24 | S-RAT (SEQ ID NO:36) | SEQ ID NO:11 | AY515122 (807-827) |
| | | SEQ ID NO:25 | | | |
| ***B. rochalimae*** | 16S-23S | SEQ ID NO:24 | S-ROC (SEQ ID NO:37) | SEQ ID NO:12 | AF415211 (414-434) |
| | | SEQ ID NO:25 | | | |
| ***Bartonella* sp*****.** ** | 16S-23S | SEQ ID NO:24 | CHOSCA (SEQ ID NO:38) | SEQ ID NO:13 | AY116639 (438-461) |
| | | SEQ ID NO:25 | | | |
| **Bartonella sp. in *Apodemus sylvaticus*** | 16S-23S | SEQ ID NO:24 | S-APO38 (SEQ ID NO:39) | SEQ ID NO:14 | AJ269792 (425-445) |
| | | SEQ ID NO:25 | | | |
| **Bartobella sp in *Meles meles*** | 16S-23S | SEQ ID NO:24 | S-TEJ (SEQ ID NO:40) | SEQ ID NO:15 | SEQ ID NO:44 (329-350) |
| | | SEQ ID NO:25 | | | |
| **Bartonella sp. i**n ***Ixodes ricinus 13*** | 16S-23S | SEQ ID NO:24 | S-G-1 3 (SEQ ID NO:41) | SEQ ID NO:16 | SEQ ID NO:45 (92-111) |
| | | SEQ ID NO:25 | | | |
| **Bartonella sp. in *Ixodes ricinus 41*** | 16S-23S | SEQ ID NO:24 | S-G-41 (SEQ ID NO:42) | SEQ ID NO:17 | SEQ ID NO:46 (86-104) |
| | | SEQ ID NO:25 | | | |

| | | | | | |
|---|---|---|---|---|---|
| ** B. chomeli*/*schoenbuchensis*/*capreoli*/*birtlesii* | | | | | |

### Brief explanation of tables 2 and 3:

▪ Column 1 (organism) indicates the bacterial species or group of species detected in each case. The *Bartonella sp.* group refers to a group of species belonging to this genus with a high degree of similarity and which are jointly detected through the method of the invention.
▪ Column 2 (gene) indicates the gene or genome region used to detect the bacterial species or group of species listed in column 1.
▪ Column 3 (primer) indicates the sequence of the pair of primers required to amplify the variable regions of the gene or intergenic space indicated in each table (column 2), in addition to the sequence in which they hybridize.
▪ Column 4 (probe) indicates the sequence of the probes used to detect the bacterial species or groups of species listed in column 1 of each table.
▪ Column 5 (sequence 5'-3') indicates the references of the sequences of the variable regions which are amplified for the detection of each bacterial species or group of species.
▪ Column 6 indicates the sequence code of a gene region or genome region listed in column 2, in addition to the specific position of each sequence in which the probe indicated in column 4 hybridizes.

### DETAILED DESCRIPTION AND EMBODIMENT METHODS

The present invention has allowed the development of an analysis method for the detection and identification of different bacterial genera and species using PCR or Multiple PCR technology. Methodology development required the analysis of intergenic space 16S-23S rRNA of gene 16S. These regions were analyzed combining different software applications and by comparison in databases, until the candidate regions susceptible to being used to apply the method were detected.

Said candidate regions were used to create a large number of primers and probes, most of which, approximately 90%, were rejected after hybridization testing, until those which did not develop crossed reactivity with samples of different origin (Figures 1 and 2, Table 5) and, additionally, had high sensitivity levels, were finally selected.

Below is a detailed description of the materials and methods used in the development of the present invention, in addition to embodiment examples thereof. Said examples do not limit the invention, but rather illustrate it, demonstrating the efficiency of the method of the invention.

### EXAMPLE 1.

### Amplification, hybridization and validation

This step includes the experimental analysis of the variable regions detected earlier using PCR for their validation. The isolated DNA was amplified using PCR, applying the following temperature cycle table and reaction mixture composition, together with the specific primers used previously for said purpose.

| Temperature Cycles | | |
|---|---|---|
| Temperature (ºC) | Time | Cycles |
| 94 | 9' | 1 |
| 94 | 15" | |
| 60 | 1' | 40 |
| 65 | 4' | |
| 65 | 7' | 1 |

PCR reaction mixture composition for a final volume of 50 µL:
- H₂O: According to final DNA volume
- Buffer Taq Gold LD: 9 µL
- Cl₂Mg [3mM]: 6 µL
- dNTPs [200mM] : 1 µL x 4
- BSA [0,8ug/uL]: 4 µL
- 8 specific Primers (SEQ ID 18-25) [50 pm/µL]: 0,5 µL of each (7 µL)
- Taq Gold LD: 0.5 µL [2.5 units]
- Problem DNA: maximum 800 ng

The amplicons were sequenced for their validation, verifying that the amplified sequence coincided with the variable sequences inferred from bioinformatic studies.

Subsequently, the amplicons were hybridized with specific probes according to the RLB protocol described by Sjoerd G. T. Rijpkema et al., Journal of Clinical Microbiology, Dec. 1995, p. 3091-3095, although applying the following modifications (Figures 1 and 2):
- Substrate: Super Signal West Dura (Pierce, Ref: 34075)
- Probes: used with a concentration of between 0.2 and 3.2 picomoles/microlitre
- Incubation: at 55ºC
- Wash: t 52ºC

Hybridization results are shown in figures 1 and 2, where we can observe that each of the probes of the invention become joined specifically to each of the amplicons of the bacterial species detected using the method of the invention.

### Preparation of samples and Multiple PCR

One of the advantages of using PCR and RLB technology-based identification systems is that pure bacterial cultures are not required. In this manner and upon validation of the primers and probes using DNA samples of the different species and subspecies listed in tables 2 and 3, a Multiple PCR-based analysis of a DNA control mixture (Figures 1 and 2) prepared under laboratory conditions was carried out, followed by a RLB test, using the specifically designed primers and probes and the previously indicated temperature cycles and reaction mixture composition.

### Detection of PCR inhibitors

An internal amplification control (IAC), which was amplified together with target DNA, was created for the detection of PCR inhibitors, using specific primers (Table 4) designed according to the conserved regions of the AB183705 sequence belonging to the THC synthase gene of the *Cannabis sativa* species. Specifically, the IAC amplicon corresponds to a sequence of 371 pairs of bases, for which a probe was also designed for detection during RLB.

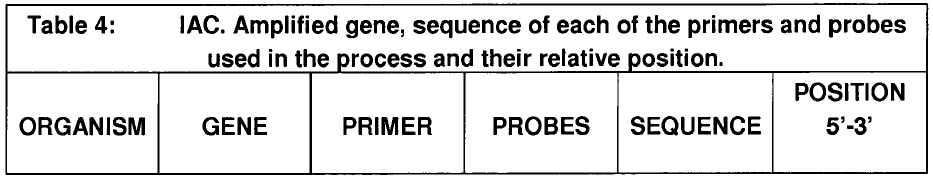

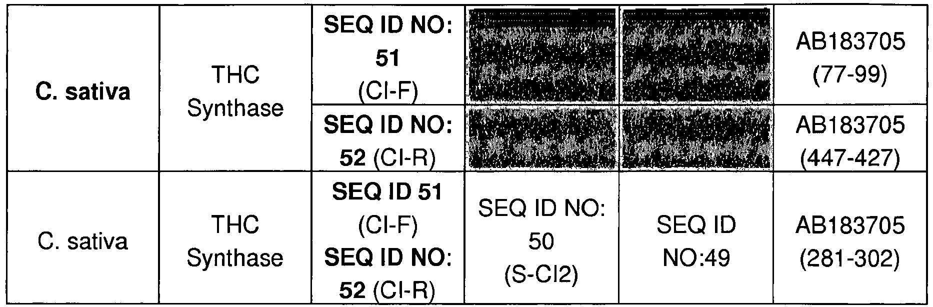

### Specificity of the method

The high specificity of this method is based on the design and selection of the primers and probes used, which were tested with another series of organisms (Table 5), following the previously described method, verifying that the formation of amplicons (Figures 1 and 2, right membrane) was in no case unspecifically detected.

| **Table 5. Specificity: unrelated bacterial, arthropod and mammal species used during method development.** | | |
|---|---|---|
| | **SPECIES** | |
| | | **RLB RESULT** |
| | **Bacteria** | |
| 1 | Brucella melitensis | Negative |
| 2 | Chlamydia penumoniae | Negative |
| 3 | Chlamydia psittaci | Negative |
| 4 | Legionella pneumophila | Negative |
| 5 | Leptospira interroqans | Negative |
| 6 | Mycoplasma pneumoniae | Negative |
| 7 | Treponema pallidum | Negative |
| 8 | Orientia tsutsugamushi | Negative |

| | **Arthropods** | Negative |
|---|---|---|
| 9 | Ixodes ricinus | Negative |
| 10 | Ripicephalus sanguineus | Negative |

| | **Mammals** | Negative |
|---|---|---|
| 11 | Apodemus sylvaticus | Negative |
| 12 | Human | Negative |

## Claims

1. Method for the simultaneous and specific identification of zoonosis-causing bacteria belonging to the genera *Anaplasma*/*Ehrlichia* and to the genus *Bartonella,* comprising the following steps:
a. Placing the sample under analysis in contact with a reaction mixture containing primers that specifically amplify the sequences SEQ ID NO: 1-7 of the *Anaplasma*/*Ehrlichia* species and sequences SEQ ID NO: 8-17 of *Bartonella* species, or their respective complementary sequences,
b. Amplifying simultaneously the sequences of step (a) by means of polymerase chain reaction,
c. Identifying the amplified sequences of step (b) by using the probes consisting of SEQ ID NO: 26-42, or their complementary sequences, to hybridise with sequences SEQ ID NO: 1-17, and
d. Detecting the presence or absence of zoonosis-causing bacteria by associating the identified sequences of step (c) to a bacterial species belonging to the genera *Anaplasma*/*Ehrlichia* or to the genus *Bartonella,*
wherein the bacterial species belonging to the genera *Anaplasma*/*Ehrlichia* are selected from the list consisting of *A. phagocytophilum, E. ruminantium, E, sennetsu, E. risticii, E. muris, A. platys, E. canis* and *E. Ovina.*

2. Method according to claim 1, in which the primers comprise the sequences SEQ ID NO: 18-25 or their complementary sequences.

3. Method according to claim 1 or 2, further comprising the primers with sequence SEQ ID NO: 51-52 in order to amplify the internal amplification control THC synthase gen of *Cannabis sativa.*

4. Method according to claim 3, in which the probes of step (c) further comprise the probe with sequence SEQ ID NO: 50 capable of hybridizing with the sequence SEQ ID NO: 49 of the internal amplification control.

5. Probes, the sequence of which consists of SEQ ID NO: 26-32 of the *Anaplasma*/*Ehrlichia* species *A. phagocytophilum, E. ruminantium, E, sennetsu, E. risticii, E. muris, A. platys, E. canis* and *E*. *ovina,* and sequences SEQ ID NO: 33-42 of *Bartonella* species, or their complementary sequences, said probes being capable of hybridizing with the sequences SEQ ID NO: 1-17 and/or with their respective complementary sequences.

6. A kit which includes probes according to claim 5.

7. The kit according to claim 6, further comprising the primers with sequences SEQ ID NO: 18-25.

8. A use of the kit according to any of claims 6 or 7, for the simultaneous and specific identification of zoonosis-causing bacteria belonging to the genera *Anaplasma*/*Ehrlichia* and to the genus *Bartonella,* wherein the bacterial species belonging to the genera *Anaplasma*/*Ehrlichia* are selected from the list consisting of *A. phagocytophilum, E. ruminantium, E, sennetsu, E. risticii, E. muris, A. platys, E. canis* and *E. Ovina.*

## Patentansprüche

1. Verfahren zur gleichzeitigen und spezifischen Identifizierung von Zoonose verursachenden Bakterien, die den Gattungen *Anaplasma*/*Ehrlichia* und der Gattung *Bartonella* angehören, das folgende Schritte umfasst:
a. Kontaktieren der zu analysierenden Probe mit einer Reaktionsmischung, die Primer enthält, die die Sequenzen SEQ ID NO: 1-7 der *Anaplasma*/ *Ehrlichia-Spezies* und die Sequenzen SEQ ID NO: 8-17 der *Bartonella*-Spezies oder ihre jeweiligen komplementären Sequenzen spezifisch amplifizieren,
b. gleichzeitiges Amplifizieren der Sequenzen aus Schritt (a) mittels der Polymerase-Kettenreaktion,
c. Identifizierung der amplifizierten Sequenzen aus Schritt (b) unter Verwendung der Sonden, die aus SEQ ID NO: 26-42 oder ihren komplementären Sequenzen bestehen, um mit den Sequenzen SEQ ID NO: 1-17 zu hybridisieren, und
d. Nachweis der An- oder Abwesenheit von Zoonose verursachenden Bakterien durch Zuordnung der identifizierten Sequenzen aus Schritt (c) einer Bakterienspezies, die den Gattungen *Anaplasma*/*Ehrlichia* oder der Gattung *Bartonella* angehört,
wobei die den Gattungen *Anaplasma*/*Ehrlichia* angehörenden Bakterienspezies aus der Liste bestehend aus *A. phagocytophilum, E. ruminantium, E. sennetsu, E. risticii, E. muris, A. platys, E. canis* und *E*. *ovina* ausgewählt werden.

2. Verfahren nach Anspruch 1, wobei die Primer die Sequenzen SEQ ID NO: 18-25 oder ihre komplementären Sequenzen umfassen.

3. Verfahren nach Anspruch 1 oder 2, das weiterhin die Primer mit der Sequenz SEQ ID NO: 51-52 umfasst, um das THC-Synthase-Gen von *Cannabis sativa* als interne Amplifikationskontrolle zu amplifizieren.

4. Verfahren nach Anspruch 3, wobei die Sonden aus Schritt (c) weiterhin die Sonde mit der Sequenz SEQ ID NO: 50 umfassen, die in der Lage ist, mit der Sequenz SEQ ID NO: 49 der internen Amplifikationskontrolle zu hybridisieren.

5. Sonden, deren Sequenz aus SEQ ID NO: 26-32 der *Anaplasma*/ *Ehrlichia-Spezies A. phagocytophilum, E. ruminantium, E. sennetsu, E. risticii, E. muris, A. platys, E. canis* und *E. ovina* und den Sequenzen SEQ ID NO: 33-42 der Bartonella-Spezies oder ihren komplementären Sequenzen besteht, wobei besagte Sonden in der Lage sind, mit den Sequenzen SEQ ID NO: 1-17 und/oder ihren jeweiligen komplementären Sequenzen zu hybridisieren.

6. Kit, das die Sonden nach Anspruch 5 beinhaltet.

7. Kit nach Anspruch 6, das weiterhin die Primer mit den Sequenzen SEQ ID NO: 18-25 umfasst.

8. Verwendung des Kits nach einem der Ansprüche 6 oder 7 zur gleichzeitigen und spezifischen Identifizierung von Zoonose verursachenden Bakterien, die den Gattungen *Anaplasma*/*Ehrlichia* und der Gattung *Bartonella* angehören, wobei die den Gattungen *Anaplasma*/*Ehrlichia* angehörenden Bakterienspezies aus der Liste bestehend aus *A. phagocytophilum, E. ruminantium, E. sennetsu, E. risticii, E. muris, A. platys, E. canis* und *E*. *ovina* ausgewählt werden.

## Revendications

1. Procédé pour l'identification simultanée et spécifique de bactéries responsables de zoonoses appartenant aux genres *Anaplasma*/*Ehrlichia* et au genre *Bartonella,* qui comprend les étapes suivantes :
a. placer l'échantillon à analyser en contact avec un mélange réactif contenant des amorces qui amplifient spécifiquement les séquences SEQ ID NO: 1-7 des espèces *Anaplasma*/*Ehrlichia* et les séquences SEQ ID NO: 8-17 de l'espèce *Bartonella,* ou leurs séquences complémentaires respectives,
b. amplifier simultanément les séquences de l'étape (a) par une réaction de polymérase en chaîne,
c. identifier les séquences amplifiées de l'étape (b) en utilisant les sondes qui comprennent SEQ ID NO: 26-42, ou leurs séquences complémentaires, à hybrider avec des séquences SEQ ID NO: 1-17, et
d. détecter la présence ou l'absence de bactéries responsables de zoonoses en associant les séquences identifiées de l'étape (c) à une espèce bactérienne appartenant aux genres *Anaplasma*/*Ehrlichia* et au genre *Bartonella,*
dans lequel les espèces bactériennes appartenant aux genres *Anaplasma*/*Ehrlichia* sont sélectionnées dans la liste composée de A. *phagocytophilum, E. ruminantium, E. sennetsu, E. risticii, E. muris, A. platys, E. canis* et *E*. *ovina.*

2. Procédé selon la revendication 1, dans lequel les amorces comprennent les séquences SEQ ID NO: 18-25 ou leurs séquences complémentaires.

3. Procédé selon la revendication 1 ou 2, comprenant en outre les amorces avec séquence SEQ ID NO: 51-52 afin d'amplifier le contrôle interne d'amplification génique THC synthase de *Cannabis sativa.*

4. Procédé selon la revendication 3, dans lequel les sondes de l'étape (c) comprennent en outre la sonde avec séquence SEQ ID NO: 50 capable d'hybridation avec la séquence SEQ ID NO: 49 du contrôle interne d'amplification.

5. Sondes, dont la séquence comprend SEQ ID NO: 26-32 des espèces *Anaplasma*/*Ehrlichia A. phagocytophilum, E. ruminantium, E. sennetsu, E. risticii, E. muris, A. platys, E. canis* et *E*. *ovina,* et les séquences SEQ ID NO: 33-42 de l'espèce *Bartonella,* ou leurs séquences complémentaires, lesdites sondes étant capables d'hybridation avec les séquences SEQ ID NO: 1-17 et/ou leurs séquences complémentaires respectives.

6. Un kit qui comprend des sondes selon la revendication 5.

7. Le kit selon la revendication 6, comprenant en outre les amorces avec séquences SEQ ID NO: 18-25.

8. Une utilisation du kit selon n'importe laquelle des revendications 6 ou 7, pour l'identification simultanée et spécifique de bactéries responsables de zoonoses appartenant aux genres *Anaplasma*/*Ehrlichia* et au genre *Bartonella,* dans laquelle les espèces bactériennes appartenant aux genres *Anaplasma*/*Ehrlichia* sont sélectionnées dans la liste composée de *A*. *phagocytophilum, E. ruminantium, E. sennetsu, E. risticii, E. muris, A. platys, E. canis* et *E*. *ovina.*


<110> Instituto de Salud Carlos III
<120> METHOD FOR THE DETECTION OF BACTERIAL SPECIES OF THE
   GENERA ANAPLASMA/EHRLICHIA AND BARTONELLA
<130> ES1613.3
<160> 52
<170> PatentIn version 3.4
<210> 1
   <211> 21
   <212> DNA
   <213> Anaplasma phagocytophylum
<400> 1
   ggtcttgaag cgctcgtaac c
   21
<210> 2
   <211> 23
   <212> DNA
   <213> Ehrlichia ruminantium
<400> 2
   gccgargcta taaataactg tcc
   23
<210> 3
   <211> 18
   <212> DNA
   <213> E. sennetsu
<400> 3
   gcaagcagct ttgattcc
   18
<210> 4
   <211> 20
   <212> DNA
   <213> E. risticii
<400> 4
   ctgcaagcag ccctgattcc
   20
<210> 5
   <211> 22
   <212> DNA
   <213> E. muris
<400> 5
   cgaacggata gctacccata gc
   22
<210> 6
   <211> 23
   <212> DNA
   <213> A. platys
<400> 6
   catagcaagc tacgacaaaa atc
   23
<210> 7
   <211> 24
   <212> DNA
   <213> E. canis/E. ovina
<400> 7
   gccagaggct ataaataatt gtcc
   24
<210> 8
   <211> 22
   <212> DNA
   <213> Genus Bartonella
<400> 8
   aatgctggca actaagggcg ag
   22
<210> 9
   <211> 24
   <212> DNA
   <213> Bartonela talpae
<400> 9
   gattaaatgg acctaaaggg actg
   24
<210> 10
   <211> 21
   <212> DNA
   <213> B. phoceensis
<400> 10
   gagagacgct tttccctttg g
   21
<210> 11
   <211> 21
   <212> DNA
   <213> B. rattimasiliensis
<400> 11
   cggtgttttg aggcaaagtg c
   21
<210> 12
   <211> 21
   <212> DNA
   <213> B. rochalimae
<400> 12
   aacagggaaa agagcaggcc a
   21
<210> 13
   <211> 24
   <212> DNA
   <213> Bartonella sp. * (B.
   chomeli/schoenbuchensis/capreoli/birtlesii)
<400> 13
   cagcaaactt atcagcaatc ataa
   24
<210> 14
   <211> 21
   <212> DNA
   <213> Bartonella sp. de Apodemus sylvaticus
<400> 14
   ccttttctcc tttttagggg c
   21
<210> 15
   <211> 22
   <212> DNA
   <213> Bartobella sp de Meles Meles
<400> 15
   gatgttttgt aaaagtgcgt cg
   22
<210> 16
   <211> 20
   <212> DNA
   <213> Bartonella sp. de Ixodes ricinus 13
<400> 16
   cgctcgtcta tcgcttgata
   20
<210> 17
   <211> 19
   <212> DNA
   <213> Bartonella sp. de Ixodes ricinus 41
<400> 17
   gcaggcactc ggcataagc
   19
<210> 18
   <211> 22
   <212> DNA
   <213> Artificial Secuence (primer)
<400> 18
   ccagcgttta gcaagataag ag
   22
<210> 19
   <211> 22
   <212> DNA
   <213> Artificial Secuence (primer)
<400> 19
   gcccagtaac aacatcataa gc
   22
<210> 20
   <211> 22
   <212> DNA
   <213> Artificial Secuence (primer)
<400> 20
   cagaacgaac gctrgcggya rg
   22
<210> 21
   <211> 22
   <212> DNA
   <213> Artificial Secuence (primer)
<400> 21
   gcrttackca cccgtctgcc ac
   22
<210> 22
   <211> 19
   <212> DNA
   <213> Artificial Secuence (primer)
<400> 22
   ccttcagttm ggctggatc
   19
<210> 23
   <211> 23
   <212> DNA
   <213> Artificial Secuence (primer)S
<400> 23
   gccyccttgc ggttagcaca gca
   23
<210> 24
   <211> 22
   <212> DNA
   <213> Artificial Secuence (primer)
<400> 24
   ttgataagcg tgaggtcgga gg
   22
<210> 25
   <211> 20
   <212> DNA
   <213> Artificial Secuence (primer)
<400> 25
   caaagcaggt gctctcccag
   20
<210> 26
   <211> 21
   <212> DNA
   <213> Artificial Secuence (probe -MSP2-)
<400> 26
   ggttacgagc gcttcaagac c
   21
<210> 27
   <211> 23
   <212> DNA
   <213> Artificial Secuence (probe -S-RUM-)
<400> 27
   ggacagttat ttatagcytc ggc
   23
<210> 28
   <211> 18
   <212> DNA
   <213> Artificial Secuence (probe -SEM-)
<400> 28
   ggaatcaaag ctgcttgc
   18
<210> 29
   <211> 20
   <212> DNA
   <213> Artificial Secuence (probe -S-RIS-)
<400> 29
   ggaatcaggg ctgcttgcag
   20
<210> 30
   <211> 22
   <212> DNA
   <213> Artificial Secuence (probe -S-MUR-)
<400> 30
   cgaacggata gctacccata gc
   22
<210> 31
   <211> 23
   <212> DNA
   <213> Artificial Secuence (probe -S-PLA-)
<400> 31
   gatttttgtc gtagcttgct atg
   23
<210> 32
   <211> 24
   <212> DNA
   <213> Artificial Secuence (probe -S-CANOVIN-)
<400> 32
   ggacaattat ttatagcctc tggc
   24
<210> 33
   <211> 22
   <212> DNA
   <213> Artificial Secuence (probe S-BARTOGEN2-)
<400> 33
   ctcgccctta gttgccagca tt
   22
<210> 34
   <211> 24
   <212> DNA
   <213> Artificial Secuence (probe -S-TOPO-)
<400> 34
   cagtcccttt aggtccattt aatc
   24
<210> 35
   <211> 21
   <212> DNA
   <213> Artificial Secuence (probe -S-PHO-)
<400> 35
   ccaaagggaa aagcgtctct c
   21
<210> 36
   <211> 21
   <212> DNA
   <213> Artificial Secuence (probe -S-RAT-)
<400> 36
   gcactttgcc tcaaaacacc g
   21
<210> 37
   <211> 21
   <212> DNA
   <213> Artificial Secuence (probe -S-ROC-)
<400> 37
   tggcctgctc ttttccctgt t
   21
<210> 38
   <211> 24
   <212> DNA
   <213> Artificial Secuence (probe -CHOSCA-)
<400> 38
   ttatgattgc tgataagttt gctg
   24
<210> 39
   <211> 21
   <212> DNA
   <213> Artificial Secuence (probe -S-AP038-)
<400> 39
   gcccctaaaa aggagaaaag g
   21
<210> 40
   <211> 22
   <212> DNA
   <213> Artificial Secuence (probe -S-TEJ-)
<400> 40
   cgacgcactt ttacaaaaca tc
   22
<210> 41
   <211> 20
   <212> DNA
   <213> Artificial Secuence (probe -S-G-13)
<400> 41
   tatcaagcga tagacgagcg
   20
<210> 42
   <211> 19
   <212> DNA
   <213> Artificial Secuence (probe -S-G-41)
<400> 42
   gcttatgccg agtgcctgc
   19
<210> 43
   <211> 452
   <212> DNA
   <213> Bartonella talpae (intergenic spacer secuence 16S-23S)
<400> 43
<210> 44
   <211> 427
   <212> DNA
   <213> Bartonella isolated from Melus Meles (intergenic
   spacer secuence 16S-23S)
<400> 44
   ttgataagcg tgaggtcgga ggttcaagtc ctcccaggcc caccaatcac actatgctga 60
   aagctcctat gattgatcgc tttttgaata agcccttaaa ggaaatttat gatcttttat 120
   aaaacttttt cccttataaa actttataaa actttcttta tgaaacttta ttgtctcaga 180
   gcattcagag agagtatgat atagcactca gaatatgata cagaaaacag agtatgagat 240
   ataaagaacg tcacctctga aattgttttt ttatcatttt aaaagtctaa aatattctgt 300
   ctctattttt aatttttaaa aagcatcaga tgttttgtaa aagtgcgtcg ttttttatag 360
   agcataacgt gaaagcattt taaactattt taggggccgt agctcagctg ggagagcacc 420
   tgctttg 427
<210> 45
   <211> 201
   <212> DNA
   <213> Bartonella isolated from Ixodes ricinus 13
   (intergenic spacer secuence 16S-23S)
<400> 45
   ttgataagcg tgaggtcgga ggttcaagtc ctccctggcc catttccttt agccccgggc 60
   tagtagctca gttggttaga tcgaggagcg acgctcgtcg atcgcttgat aagcgtgagg 120
   tcggaggttc aagtcctccc aggcccacca tattcctata ccttacgggg gcgtagctca 180
   gctgggagag cacctgcttt g 201
<210> 46
   <211> 186
   <212> DNA
   <213> Bartonella isolated from Ixodes ricinus 41
   (intergenic spacer secuence 16S-23S)
<400> 46
<210> 47
   <211> 21
   <212> DNA
   <213> Genus Anaplasma/Ehrlichia
<400> 47
   catgcaagtc gaacggatta t 21
<210> 48
   <211> 20
   <212> DNA
   <213> Genus Anaplasma/Ehrlichia (probe-AE-GEN-)
<400> 48
<210> 49
   <211> 22
   <212> DNA
   <213> Cannabis sativa (THC sintase)
<400> 49
   cctcctccac taaagtgtcc ac 22
<210> 50
   <211> 22
   <212> DNA
   <213> Artificial Secuence (probe -S-CI-2-)
<400> 50
   gtggacactt tagtggagga gg
   22
<210> 51
   <211> 23
   <212> DNA
   <213> Artificial Secuence (primer, CI-F)
<400> 51
   atgatgctga gggtatgtcc tac
   23
<210> 52
   <211> 21
   <212> DNA
   <213> Artificial Secuence (primer, CI-R)
<400> 52
   gttttctcct ccaccaccac g
   21